(19) 

**Europäisches Patentamt**

**European Patent Office**

**Office européen des brevets**

(11)  **EP 4 686 759 A1**

(12) **EUROPEAN PATENT APPLICATION**

(43) Date of publication:
04.02.2026 Bulletin 2026/06

(21) Application number: 24191820.0

(22) Date of filing: 30.07.2024

(51) International Patent Classification (IPC):
***C12P 5/02*** *(2006.01)*

(52) Cooperative Patent Classification (CPC):
**C12P 5/023; C12M 21/04;** C12C 1/033;
C12C 1/125; C12M 43/08

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **WCHF Europe j. s. a.**
**841 03 Bratislava - mestská cast Lamac (SK)**

(72) Inventors:
• **Michalíková, Petronela**
**900 31 Stupava (SK)**
• **Halabica, Jakub**
**900 31 Stupava (SK)**

(74) Representative: **inventa Patentova a znamkova
kancelaria s.r.o.
Palisády 50
811 06 Bratislava (SK)**

(54) **BIOFUEL PRODUCTION METHOD USING OPERATIONALLY INTERCONNECTED GERMINATION AND BIOGAS PLANT SUBSYSTEMS, AND THE GERMINATION PLANT SUBSYSTEM**

(57) The biofuel production method comprises a series of steps performed in the germination plant (2) subsystem and operationally interconnected biogas plant (3). The process starts with the provision of grains, followed by separation of impurities and soaking in water. The grains are then germinated with controlled irrigation and exposure to light. After germination, the grains are mixed and homogenised to form biofuel (1) for the biogas plant (3). This biofuel (1) is then used to produce biogas (6). The method includes embodiments such as using specific types of grain, mixing with broken parts of the grain or unused water, and using biogas plant (3) co-products such as fugate (4) as fertilizer, and residual energy (7) for temperature control. In addition, the method includes the production of biogas (6) and electric energy (8), wherein the electricity (8) is potentially used to illuminate the grain during the germination.

Fig. 1

## Description

### Field of the invention

The present invention relates to the method of biofuel production using operationally interconnected germination plant subsystem and biogas plant subsystem, whereby the biofuel production is performed in the closed self-sufficient cycle, which enables full utilization of the installed capacity of the biogas plant, thereby achieving environmentally friendly production of renewable energy. The invention falls within the field of energy.

### Background of the invention

The support of renewable sources, including biogas plants (BPS), has emerged as an economically interesting alternative for the utilisation of surplus agricultural production. It is known from the state of the art that, in general, biogas plants can be divided into agricultural and waste biogas plants. In agricultural biogas plants, targeted energy crops such as oilseed rape or maize are often used for biogas production in addition to agricultural waste. However, a large number of inputs are needed for their production and processing, ranging from fertilisers and pesticides up to diesel as fuel for tractors.

In waste biogas plants, the input raw material is any organic material. These are mainly animal faeces or residual biomass from agricultural production. However, the construction of waste biogas plants faces higher investment costs due to the need of hygiene of a waste. The problem that biogas plants currently face is the lack of ability to provide sufficient raw material to keep the biogas plant running smoothly. This leads to a dependence on suppliers for the raw material for operation of the biogas plant.

US patent application US20090215141A1 describes an anaerobic digestion device that is operationally coupled to a hydroponic system. The hydroponic system is used to grow energy crops that are used as biofuel for the digester. The hydroponic subsystem is connected to a so-called agricultural type of digester, which uses purposely grown energy crops rather than waste agricultural raw material which can no longer be used for food purposes as is the case with present subject of the patent application.

Document WO2017141178A1 describes the method and system to increase the area for targeted cultivation of energy crops to be used for biogas production. The system comprises the rotating growth pad, wherein the rotating growth pad is suspended vertically on the roller system. The above mentioned document addresses the problem of the lack of soil for the cultivation of energy crops as biofuel for the production of biogas and, consequently, methane, given that agricultural soil is in decline and should primarily be used for the cultivation of food crops. The document does not mention the use of energy crops, which are waste and thus have no use as food or feed.

### Summary of the invention

In agricultural biogas plants, targeted energy crops such as oilseed rape or maize are often used to produce biogas in addition to agricultural waste. However, a large number of inputs are needed for their production and processing, ranging from fertilisers and pesticides up to diesel as fuel for tractors.

In compliance with the above mentioned shortcomings in the state of the art, there is a need to provide the biogas plant with a self-sufficient and environmentally friendly supply of its own biofuel, which is not primarily intended for food and agricultural purposes. In an effort to solve this challenge, the biofuel production method has been proposed using operationally interconnected germination and biogas plant subsystems.

According to the first aspect of the present invention, therefore, an economically advantageous and environmentally friendly method of biofuel production is provided, utilizing operationally interconnected germination and biogas plant subsystems. Such interconnection allows exploit the synergy between the germination plant, which produces a sufficient amount of biofuel or feed required for the continuous production of biogas, and the biogas plant, which provides the necessary carbon dioxide, energy in the form of heat and light as well as additional nutrients to accelerate the germination process. The advantage of such a synergistic operational link is also the fact that the need for additives to stabilize the processes in the biogas plant subsystem is minimized, both because the germination subsystem continuously supplies the necessary amount of feed in constant quality, as well as because part of the feed or biofuel is preferably also water, not consumed during soaking or germination, the quality and composition of which is at the level of molasses and therefore contains the necessary additives.

The germination process in the germination plant begins with the stacking and weighing of the required daily batch of grains, which are conveyed via conveyors to separate impurities and broken parts of the grains into the sorting device. Subsequently, the good grains are separated from the damaged grains to ensure the optimum characteristics of the biofuel produced and its efficiency. The undamaged separated grains are subjected to soaking and the fractured parts are crushed, preferably to powder, preferably also to be used subsequently in the production of biofuel.

This sorting process produces no waste, so the entire input material is used. Because of the advantageous relationship between germination and relative methane production capacity during the putrefaction process, it is advantageous to use grains with the ratio (i.e. EGC, EGCk) between relative methane production (met, standard cubic meter, $Nm^3$) and grain germination (kl, the ratio between the grain's ability to increase its weight (m) in a given number of days of germination (tkl)) of less than 200. It is particularly advantageous to use rye, which has the lowest ratio of the common grains.

In the method according to the invention, it is also possible to make advantageous use of low-quality grains, for example, half-rotten or contaminated grains, which cannot be used for food, feed or agricultural purposes.

In the next step, soaking is performed in tanks or silos where the grain is fully submerged for a set soaking time in water. During soaking, it may advantageously be stirred or aerated to prevent the grains from sticking together and forming clumps. During soaking, the grain germinates in preparation for the subsequent steps in the germination process. The water in which the grain is soaked is conveniently not diverted outside the germination subsystem, but is used in the subsequent steps of the biofuel (EGC) production, which makes it possible to ensure a high level of environmental sustainability of the whole process and to accelerate the germination process thanks to the nutritional value of such water, which is at the level of molasses. In order to speed up the subsequent germination, it is preferable that the soaking time during which the grain germinates to the required form is at least 12 hours. Preferably, the soaking time is a maximum of 24 hours, as it is not efficient to soak the grain longer because longer soaking will not accelerate the quality and speed of subsequent germination.

The batched volume of soaked germinated grains is evenly distributed mechanically or preferably automatically onto the germination trays. Subsequently, the germination trays are conveniently stacked in germination baskets and said germination baskets are further conveniently placed in racks, which represent a vertical warehouse racking system in the germination plant, optimizing logistic operations and allowing a high degree of automation thereof. In the racks, the process of germination of the germinated grains is carried out during the germination period, with simultaneous irrigation and illumination, allowing the germination process to be performed. The germination time depends on the grain used and preferably is not less than 5 days in order to generate sufficient organic matter for the efficiency of the process. It is also preferable that the germination process is terminated when the peak germination is reached, at which point the effective germination dynamization, i.e. the daily weight gain of organic matter, begins to slow down. It is therefore preferable to use grains that reach peak germination after 6 days, such as rye, which allows an optimal seven-day operation cycle of the germination plant subsystem to be selected, where one new daily batch of grain is batched and soaked each day, and one daily batch of biofuel is removed from the oldest daily batch of grain in the germination plant subsystem. For reasons of efficiency, it is also advantageous to select grains that reach peak germination after a maximum period of 8 days.

The germination plant preferably maintains a microclimate providing optimal conditions for germination, and it is therefore advantageous if the germination plant is an enclosed building in which suitable conditions for germination and biofuel production can be ensured, regardless of weather conditions and season of the year. It is advantageous to use the energy generated during the production of biogas in the interconnected biogas plant, such as residual heat or the biogas itself for the needs of heating the germination plant building as well as the generated electric energy, which will be used in the illumination of the germinating grains by light radiation, in the creation of such a microclimate. In this way, it is possible to achieve a high degree of energy independence of the germination plant subsystem from external energy sources, as well as to increase the economic efficiency of the biogas and energy production process. The enclosed object may advantageously be the hall.

According to the present invention, it is advantageously possible to operatively connect a plurality of germination plant subsystems with a single biogas plant, a plurality of biogas plants with a single germination plant subsystem as well as a plurality of germination plant subsystems with a plurality of biogas plants for spatial or operational optimization.

During germination, it is advantageous to use the nitrogen liquid fugate from anaerobic fermentation in the biogas plant to further stimulate the germination process as a fertilizer, which is preferably fed to the germinating grains together with irrigation. This use of fugate is made possible by the operational interconnection of the germination and biogas plant subsystems and, in addition to further increasing the efficiency of the germination process and biogas production, also contributes to the sustainability of the overall biogas production process.

Once the desired level of germination is reached, the germinated grains are removed from the germination baskets and trays and expanded into a tank, where they are conveniently mixed with the crushed fracture waste grains and the unabsorbed water from the soaking and germination process. These steps allow for optimum raw material utilization as well as sustainability of the entire biofuel production process and, due to the use of non-consumed water, minimize the need for additional additives to stabilize the processes in the biogas plant subsystem.

Crushing and mixing produces a homogeneous biofuel mixture (Eco Green Circuit, EGC) for the biogas plant subsystem (BPS), thus ensuring an optimal operating mode.

This is followed by the process of "feeding" the biogas plant. In the method according to the present invention, the conventional biogas plant well known to one skilled in the art is used. The biofuel is conveyed from the germination subsystem to the homologation sump (older types of BPS) or to the fermenters of the biogas plant, where decomposition and production of biogas and energy is realized with the manner known in the art. Preferably, pumps are used for transport. Preferably, the secondary fermenter is also included in the BPS subsystem, in which case multi-stage digestion is performed.

The fermented substrate is stored in the final warehouse. The biogas produced in the biogas plant by anaerobic fermentation can be further advantageously used in the cogeneration unit to generate electricity and heat, which are advantageously used to heat or cool the enclosed germination building (i.e. the germination room) and also to illuminate the grains. The biogas can be further purified in the purifier up to biomethane.

Another product of biogas production is digestate, which can be used as such, for example, as the organic fertiliser, or further processed into the liquid part (water enriched with nutrients) called fugate. The fugate is filtered and preferably returned to the germination plant and used for irrigation during germination and possibly also for soaking the grain. Fugate as the waste product of the production process is thus advantageously used in the germination plant to dynamise growth.

According to the second aspect of the present invention, the germination plant subsystem is provided, which is preferably the large capacity hall (EGC hall) designated for germination ensuring the needs of the biogas plant in terms of raw material input and the capacity is flexibly scaled to use 100% of the installed capacity of the biogas plant.

The germination plant subsystem has the means to create its own microclimate and the means to illuminate the germinating grains with light radiation, the necessary energy being obtained directly from the operationally interconnected biogas plant. This makes the germination plant subsystem energy independent from external energy sources. Furthermore, the germination subsystem includes all the means for ensuring the soaking and germination of the grain, including germination trays, baskets and racks and preferably also means for automation of the handling thereof.

In practice, the germination plant subsystem, as referred to in this description is the substitute for the homologation sump or feeding basket. The biofuel is transported directly from the germination plant (ECG hall) to the fermenters. This creates an operationally interconnected unit comprising the germination subsystem and the biogas plant, with biofuel in one direction and energy, carbon dioxide as well as nitrogen nutrients in the other direction, enabling the optimisation of the germination process. Such closed system eliminates the need for external biofuel and external energy sources, while allowing continuous operation independent of weather conditions, season and agrotechnical cycle.

In accordance with the third aspect of the present invention application, an EGC coefficient (Eco Green Circuit coefficient, EGCk) is provided. The EGC coefficient takes into account the set of properties of the input raw material, grain. More specifically, it preferably relates to cereals such as wheat, barley, rye, for example, which can multiply their weight in a few days and whose growth can be very well supported by means of artificial lighting and circulated irrigation (water enriched with nutrients, preferably fugate).

The above mentioned set of grain properties includes germination rate (kl), number of days of germination (tkl), the relative amount of methane produced during the putrefaction process from 1 tonne of germinated grain (met, given in standard cubic metres $Nm^3$; this quantity is measurable by instruments known to the expert).

The relative germination (kl) is the ability of the grain to increase in weight (m, in tonnes) given the number of days of germination (tkl) to reach peak germination at the input weight of 1 tonne before the start of germination and it is given by the equation:

$$\mathbf{kl = m : tkl}$$

The EGC coefficient is then defined as the methane production (met) relative to the germination rate (kl) and it is given by the equation:

$$\mathbf{EGCk = met : kl.}$$

The coefficient (EGCk) makes it possible to accurately determine the profitability of specific grains for use in biogas plants. The lower the EGC coefficient (EGCk), the more cost-effective and resource-efficient the particular EGC fuel is.

Thus, the EGC coefficient makes it possible to assess the suitability of the given grain for its use in the biofuel production method using operationally interconnected germination and biogas plant subsystems according to the present invention.

**Overview of images on drawings**

The method of biofuel production using the operationally interconnected germination plant subsystem and the biogas plant subsystem, so that the biofuel production is performed in the closed self-sufficient cycle, will be further explained in the drawings on which:

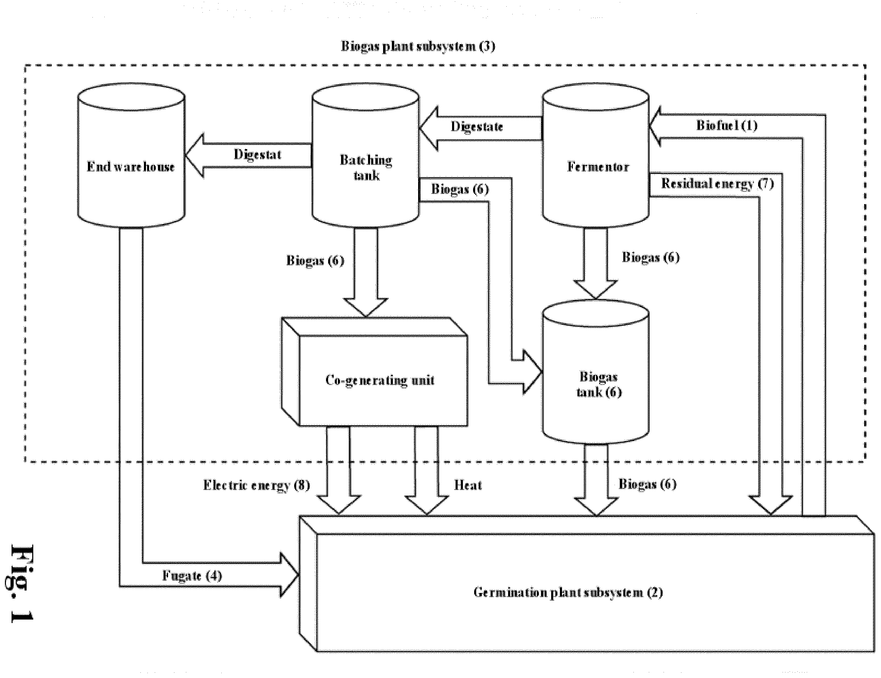

FIG. 1 illustrates the diagram of interconnection of the germination plant subsystem and the biogas plant subsystem according to embodiments of the present invention.

**Examples of embodiments of the invention**

It is understood that the various embodiments of the invention are presented for purposes of illustration and not as limitations of solutions.

Example 1

The batch of 1 tonne of rye grain was fed through conveyors into the sorting device. Impurities and broken grains were separated from the batch. The undamaged grains were placed into the silo located in the germination plant 2 subsystem, in which they were immersed in the incoming water and soaked for 12 hours with periodic mixing. During this time the grains were germinated.

Subsequently, the soaked germinated grain was rolled into germination trays which were stacked into germination baskets placed in racks that were located in the dedicated section of germination plant 2. During the germination period of 6 days, the grains were regularly irrigated with water, mixed with fugate 4, from the operatively interconnected biogas plant 3, using also the unconsumed water from the soaking. The grain was illuminated by light at regular intervals and in germination plant 2, where room temperature was maintained at a constant humidity during this time. The energy generated in the interconnected biogas plant 3 was used to maintain the microclimate.

After period of 6 days, the germinated grain was removed from the germination baskets and transferred by conveyor to the tank where it was mixed with the fractured grains from the sorting process and the unconsumed waste water from the soaking and germination process to form a homogeneous mixture of biofuel 1 (EGC biofuel) for the subsystem of the operationally interconnected biogas plant 3.

Subsequently, the biofuel 1 thus formed was transferred by means of pumps from the tank in the germination plant 2 to the fermenter 5 of the operatively interconnected biogas plant 3.

Example 2

The separate 1 tonne batches of wheat, barley and rye were separated and soaked in the manner described in Example 1. Subsequently, then each soaked germinated grain was rolled into germination trays which were stacked into germination baskets placed in racks which were located in the dedicated section of germination plant 2. During the germination period, the grains were regularly irrigated with water. The grains were illuminated by light radiation at regular intervals and during this period the room temperature as well as the humidity was maintained at the constant level in germination plant 2.

On the daily basis, the relative daily weight gain of the soaked grain was measured by sampling, whereby germination for the given grains was terminated when it reached its peak, and hence the relative daily weight gain started to slow down.

Subsequently, the total weight of such germinated grain was measured, its relative germination rate was calculated, and its relative methane production during the putrefaction process was determined using the BioReactor Simulator from BPC Instruments and the EGC coefficient was determined with its help. The results are presented in Table 1.

Table 1

|  | WHEAT | BARLEY | RYE |
|---|---|---|---|
| Weight of germinated grains after given number of days of germination (**m**, t) | 5 | 7 | 8 |
| Number of germination days to peak germination (**tkl**) | 7 | 7 | 6 |
| Relative germination (**kl**) | 0,714 | 1,000 | 1,167 |
| Relative methane formation during the putrefaction process (**met,** $Nm^3$) | 170 | 160 | 180 |
| EGC coefficient (**EGCk**) | 238 | 160 | **154** |

**Industrial applicability**

The biofuel production method using operatively interconnected germination plant subsystems and biogas plant subsystems of the germination plant subsystem for its implementation according to the present invention can be used in the field of power engineering.

**List of reference numbers**

1    biofuel
2    germination plant - subsystem
3    biogas plant - subsystem -
4    fugate
5    fermenter
6    biogas
7    residual energy
8    electric energy

**Claims**

1.  A method for producing biofuel (1) using operationally interconnected germination plant (2) and biogas plant (3) subsystems, comprising the following steps performed in the germination plant (2) subsystem:

    i. providing the necessary batch of grains;
    ii. separation of impurities and broken parts of grains in a sorting device;
    iii. soaking the separated grains from step (ii) in water for the soaking period, with simultaneous stirring;
    iv. batching the soaked grains from step (iii) onto a germination trays;
    v. stacking the germination trays containing the germinated grains from step (iv) into a germination baskets;
    vi. placing the germination baskets with the stacked germination trays from step (v) in a germination racks located in the enclosed germination object;
    vii. germination of the soaked grains in the germination racks from step (vi) for the germination period of at least 5 days with simultaneous irrigation with water, and light illumination;
    viii. removing the germinated grains from step (vii) from the germination baskets and trays and placing them into a collection tank for feed;
    ix. mixing the germinated grains from step (vii) in the collection tank for feed to produce a mixture of germinated grains;
    x. homogenisation of the mixture from step (ix) to produce the biofuel (1) for the biogas plant (3) subsystem;
    as well as the following steps:
    xi. transfer of the biofuel (1) from step (x) to the homologation sump or fermenter (5) of the biogas plant (3) subsystem;
    xii. production of the biogas (6) from biofuel (1) from step (xi) in the biogas plant (3) subsystem.

2.  The method according to claim 1, **characterized in that** the soaking time is at least 12 hours.

3.  The method according to any one of the previous claims, **characterized in that** the soaking time is 24 hours maximum.

4.  The Method according to any one of the previous claims, **characterized in that** the germination period is at least 5 days.

5.  The method according to any one of the previous claims, **characterized in that** the germination period is the maximum of 8 days.

6.  The method according to any one of the previous claims, **characterized in that** water not consumed in step (iii) is used for irrigation in step (vii);

7.  The method according to any one of the previous claims, **characterized in that** the grains have the ratio between the relative amount of methane produced per tonne of grain during the putrefaction process and the relative germination of the grains maximum 200.

8.  The method according to claim 7, **characterized in that** the grain is rye.

9. The method according to any one of the previous claims, **characterized in that** the germination period is 6 days.

10. The method according to any one of the previous claims, **characterized in that** in step (ix) the germinated grains are mixed with the broken grain portions separated in step (ii).

11. The method according to any one of the previous claims, **characterized in that** in step (ix) the germinated grains are mixed with water not consumed in step (iii) or step (vii).

12. The method according to any one of the previous claims, **characterized in that** the fugate (4) produced in the biogas plant (3) subsystem from the biofuel (1) produced in step (x) is used as fertilizer in the germination of the soaked grains in step (vii).

13. The method according to any one of the previous claims, **characterized in that** the residual energy (7) generated in the biogas plant (3) subsystem in step (xii) is used to control the temperature in the germination plant (2) subsystem.

14. The method according to any one of the previous claims, **characterized in that,** in the biogas plant (3) subsystem, biogas (6) and electric energy (8) are produced from the biofuel (1) produced in step (x).

15. The method according to claim 14, **characterized in that** the electrical energy (8) produced from the biofuel (1) in the biogas plant (3) subsystem is used in the illumination of the soaked grains by light radiation during their germination in the germination plant (2) subsystem in step (vii), or in the control of the temperature in the germination plant (2) subsystem.

16. A germination plant (2) subsystem, comprising means for performing the method according to any one of the previous claims.

17. The germination plant (2) subsystem according to claim 16, **characterized in that** the enclosed germination object is the hall.

Fig. 1

Biogas plant subsystem (3)

End warehouse

Batching tank

Fermentor

Digestat

Digestate

Biofuel (1)

Biogas (6)

Residual energy (7)

Biogas (6)

Biogas (6)

Co-generating unit

Biogas tank (6)

Electric energy (8)

Heat

Biogas (6)

Fugate (4)

Germination plant subsystem (2)

EP 4 686 759 A1

**Europäisches Patentamt**
**European Patent Office**
**Office européen des brevets**

# EUROPEAN SEARCH REPORT

**Application Number**

EP 24 19 1820

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2009/020741 A1 (TATE AND LYLE INGREDIENTS AMER [US]; KERR JOHN [GB] ET AL.) 12 February 2009 (2009-02-12) * claims 1,2 * | 1-15 | INV. C12P5/02 |
| A | HUTNAN MIROSLAV ET AL: "Biogas Production from Maize Grains and Maize Silage", POLISH JOURNAL OF ENVIRONMENTAL STUDIES, vol. 9, no. 2, 24 September 2010 (2010-09-24), pages 323-329, XP093235254, * Abstract; Experimental section, 2nd and 4th paragraph, page 324; Results section 4th paragraph, page 327 * | 1-15 | |
| A | WO 2004/016796 A1 (TEKNISKA VERKEN LINKOEPING AB [SE]; HOLM STIG [SE] ET AL.) 26 February 2004 (2004-02-26) * claim 1; examples 1,3; table 2 * | 1-15 | |
| X | GB 915 071 A (GEORGE TWEEDY AND COMPANY LTD) 9 January 1963 (1963-01-09) * claims 1,2,6 * | 16,17 | **TECHNICAL FIELDS SEARCHED (IPC)** |
| X | US 3 965 614 A (KIENHOLZ KARL E) 29 June 1976 (1976-06-29) * claim 1 * | 16,17 | C12P C12M C12C |

The present search report has been drawn up for all claims

| Place of search | Date of completion of the search | Examiner |
|---|---|---|
| The Hague | 23 December 2024 | Blanco Parte, F |

EPO FORM 1503 03.82 (P04C01)

## ANNEX TO THE EUROPEAN SEARCH REPORT
## ON EUROPEAN PATENT APPLICATION NO.

EP 24 19 1820

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2024

| Patent document cited in search report | | Publication date | Patent family member(s) | | Publication date |
|---|---|---|---|---|---|
| WO 2009020741 | A1 | 12-02-2009 | AR | 067872 A1 | 28-10-2009 |
| | | | EP | 2181194 A1 | 05-05-2010 |
| | | | US | 2009095286 A1 | 16-04-2009 |
| | | | WO | 2009020741 A1 | 12-02-2009 |
| WO 2004016796 | A1 | 26-02-2004 | AU | 2003237758 A1 | 03-03-2004 |
| | | | EP | 1537220 A1 | 08-06-2005 |
| | | | SE | 522262 C2 | 27-01-2004 |
| | | | US | 2006102560 A1 | 18-05-2006 |
| | | | WO | 2004016796 A1 | 26-02-2004 |
| GB 915071 | A | 09-01-1963 | NONE | | |
| US 3965614 | A | 29-06-1976 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090215141 A1
- WO 2017141178 A1